# EUROPÄISCHE PATENTANMELDUNG

(11) **EP 2 543 395 A1**
(43) Veröffentlichungstag der Anmeldung: **09.01.2013**
(21) Anmeldenummer: 11172566.9
(22) Anmeldetag: 04.07.2011
(51) Int. Cl.: A61L 24/04, A61L 31/06

(54) **Schichtaufbau zum Verschließen von Gewebeleckagen**

(71) Anmelder: Bayer MaterialScience AG, 51373 Leverkusen (DE)
(72) Erfinder: Eggert, Christoph, Dr., 50733 Köln (DE); Heckroth, Heike, Dr., 51519 Odenthal (DE)

(57) **Zusammenfassung**

Die vorliegende Erfindung betrifft einen Schichtaufbau, insbesondere zum Verschließen von Gewebeleckagen mit einer Trägerfolie und einer Klebstoffschicht, wobei die Klebstoffschicht zumindest bereichsweise mit der Trägerfolie verbunden ist und die Klebstoffschicht ein Gemisch wenigstens folgender Komponenten umfasst: Ein isocyanatfunktionelles Prepolymer erhältlich durch Umsetzung von wenigstens einem aliphatischen Polyisocyanat mit einem Polyol, das insbesondere ein zahlenmittleres Molekulargewicht von ≥ 400 g/mol und eine mittlere OH-Funktionalität von 2 bis 6 aufweisen kann und einer aminofunktionellen Verbindung der allgemeinen Formel (I)

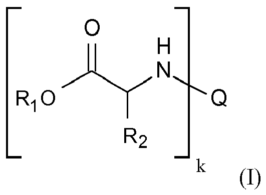

in der R₁ ein organischer Rest, der keinen Zerewitinoff-aktiven Wasserstoff aufweist, R₂ ein linearer oder verzweigter, unsubstituierter oder mit Heteroatomen auch in der Kette substituierter organischer Rest ist, der keinen Zerewitinoff-aktiven Wasserstoff aufweist, Q ein linearer oder verzweigter, unsubstituierter oder mit Heteroatomen auch in der Kette substituierter organischer Rest und k ≥ 1 und ≤ 6 ist.

Weitere Gegenstände der Erfindung sind ein Verfahren zur Herstellung des erfindungsgemäßen Schichtaufbaus sowie ein Kit zur Herstellung eines erfindungsgemäßen Schichtverbunds.

## Beschreibung

Die vorliegende Erfindung betrifft einen Schichtaufbau, insbesondere zum Verschließen von Gewebeleckagen. Weitere Gegenstände der Erfindung sind ein Verfahren zur Herstellung des erfindungsgemäßen Schichtaufbaus sowie ein Kit zur Herstellung eines erfindungsgemäßen Schichtverbunds.

Im Stand der Technik sind verschieden Arten von Gewebekleber beschrieben, die teilweise auch zum Verschließen von Gewebeleckagen verwendet werden können. Insbesondere dann, wenn aus den Gewebeleckagen Fluide wie Gase oder Flüssigkeiten mit einem gewissen Druck ausströmen, ist mit den bekannten Systemen häufig kein Verschluss möglich. So kann der Gewebekleber entweder bereits nicht mit der nötigen Präzision aufgetragen werden oder er wird von dem ausströmenden Fluid verdrängt, bevor er ausgehärtet ist.

Aufgabe der Erfindung war es daher ein System bereit zu stellen, mit dem einfach, schnell, sicher und zuverlässig auch Gewebeleckagen, aus denen ein Fluid austritt, verschlossen werden kann.

Diese Aufgabe ist durch einen Schichtaufbau mit einer Trägerfolie und einer Klebstoffschicht gelöst bei dem die Klebstoffschicht zumindest bereichsweise mit der Trägerfolie verbunden ist und die Klebstoffschicht ein Gemisch wenigstens folgender Komponenten umfasst:
A) ein isocyanatfunktionelles Prepolymer erhältlich durch Umsetzung von wenigstens
   A1) einem aliphatischen Polyisocyanat mit
   A2) einem Polyol, das insbesondere ein zahlenmittleres Molekulargewicht von > 400 g/mol und eine mittlere OH-Funktionalität von 2 bis 6 aufweisen kann,
B) eine aminofunktionelle Verbindung der allgemeinen Formel (I) 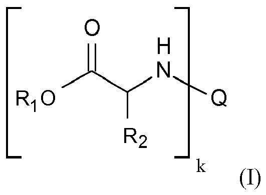
in der
- R₁: ein organischer Rest, der keinen Zerewitinoff-aktiven Wasserstoff aufweist,
- R₂: ein linearer oder verzweigter unsubstituierter oder mit Heteroatomen auch in der Kette substituierter organischer Rest ist, der keinen Zerewitinoff-aktiven Wasserstoff aufweist,
- Q: ein linearer oder verzweigter, unsubstituierter oder mit Heteroatomen auch in der Kette substituierter organischer Rest und
- k: ≥ 1 und ≤ 6
ist.

Zur Definition von Zerewitinoff-aktivem Wasserstoff wird auf Römpp Chemie Lexikon, Georg Thieme Verlag Stuttgart verwiesen. Bevorzugt werden unter Gruppen mit Zerewitinoff-aktivem Wasserstoff OH, NH oder SH verstanden.

Gemäß einer ersten bevorzugten Ausführungsform ist R₁ ein C1 bis C10, bevorzugt ein C1 bis C8, besonders bevorzugt ein C2 bis C6 Alkyl-Rest und ganz besonders bevorzugt ein Methyl-und / oder ein Ethyl-Rest.

Bevorzugt ist auch, wenn Q ein linearer oder verzweigter, unsubstituierter oder mit Heteroatomen auch in der Kette substituierter C1 bis C10, bevorzugt C2 bis C8, besonders bevorzugt C3 bis C6 Rest ist.

Gemäß einer weiteren bevorzugten Ausführungsform ist vorgesehen, dass Q eine (CH₂)ₚ-O-Gruppe mit p ≥ 2 ≤ 4 ist oder ein CH₂-Gruppe umfasst.

Besonders bevorzugt ist k ≥ 1 und ≤ 4 und weiter bevorzugt ≥ 2 und ≤ 3.

Es ist ebenfalls möglich, dass Q insbesondere in der Kette eine Acetalgruppe aufweist. Dies führt dazu, dass das resultierende System noch schneller abbaubar ist.

Besonders bevorzugt ist daher eine aminofunktionelle Verbindung B) der Formel (II) 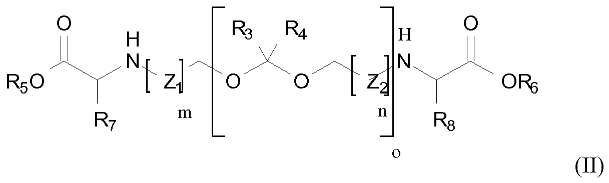 gelöst, in der
- R₃: ein C1- bis C6-Alkyl-Rest oder H ist,
- R₄: ein organischer Rest enthaltend eine sekundäre Aminofunktion ist,
- R₅, R₆: jeweils unabhängig voneinander ein organischer Rest sind, der keinen Zerewitinoff-aktiven Wasserstoff aufweist,
- R₇, R₈: jeweils unabhängig voneinander ein CH₂-COOR₉-Rest, bei dem R₉ ein organischer Rest ist, der keinen Zerewitinoff-aktiven Wasserstoff aufweist, ein linearer oder verzweigter C1- bis C4-Alkyl-, ein Cyclopentyl-, ein CyclohexylRest oder H sind,
- Z₁, Z₂: jeweils unabhängig voneinander ein (CH₂)ₐ₋O-Rest, wobei a ≤ 2 ≤ 4 ist, oder ein CH₂-Rest sind,
- n, m: jeweils unabhängig voneinander ≥ 1 und ≤ 6 sind und
- o: ≥ 1 und ≤ 10 ist.

Ganz besonders bevorzugt ist hier, wenn R₅ ein Rest der Formel (III) 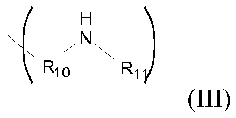 ist, in der
- R₁₀, R₁₁: jeweils unabhängig voneinander ein organischer Rest sind, der keinen Zerewitinoff-aktiven Wasserstoff aufweist.
R₁₀ kann ein linearer oder verzweigter, gesättigter, gegebenenfalls auch in der Kette mit Heteroatomen substituierter organischer Rest, insbesondere ein linearer oder verzweigter, gesättigter, aliphatischer C1 bis C10, bevorzugt C2 bis C8 und besonders bevorzugt C2 bis C6 Kohlenwasserstoffrest sein.

R₁₁ kann dabei vorzugsweise ein Rest der Formel (IV) 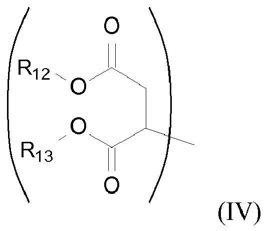 sein, in der
- R₁₂, R₁₃: jeweils unabhängig voneinander oder gleichzeitig ein C1- bis C10, bevorzugt ein C1 bis C8, besonders bevorzugt ein C2 bis C6 AlkylRest und ganz besonders bevorzugt ein Methyl und / oder ein EthylRest sind.

Bevorzugt ist auch, wenn in der Formel (III) R₇, R₈ jeweils ein CH₂-COOR₉-Rest sind, bei dem R₉ ein organischer Rest ist, der keinen Zerewitinoff-aktiven Wasserstoff aufweist.

R₆, R₇ und gegebenenfalls R₉ können jeweils unabhängig voneinander oder gleichzeitig ein C1-bis C10, bevorzugt ein C1 bis C8, besonders bevorzugt ein C2 bis C6 Alkyl-Rest und ganz besonders bevorzugt ein Methyl und / oder ein Ethyl-Rest sind.

Bevorzugt ist auch, wenn Z₁ und Z₂ jeweils gleichzeitig ein CH₂-Rest sind.

Die Indizes n und m können jeweils unabhängig voneinander oder gleichzeitig > 1 und < 6, bevorzugt > 1 und < 4 und besonders bevorzugt 1 oder 2 sein.

Ebenfalls vorteilhaft ist, wenn o ≥ 1 und ≤ 10, bevorzugt ≥ 1 und ≤ 4 und besonders bevorzugt 1 oder 2 ist.

Vorteilhaft ist auch, wenn Q insbesondere in der Kette eine sekundäre Aminofunktion aufweist. Dies führt dazu, dass das System besonders schnell aushärtet.

Besonders vorteilhaft ist daher eine aminofunktionelle Verbindung der Formel (V) 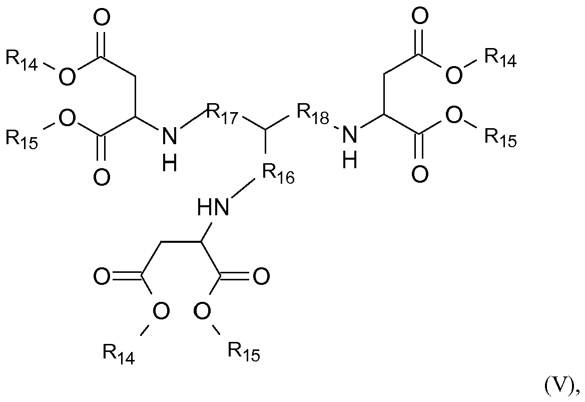 ,in der
- R₁₄, R₁₅: jeweils unabhängig voneinander gleiche oder verschiedene organische Reste, die keinen Zerewitinoff-aktiven Wasserstoff aufweisen und
- R₁₆, R₁₇, R₁₈: jeweils unabhängig voneinander gesättigte, linearer oder verzweigter, gegebenenfalls auch in der Kette mit Heteroatomen substituierte, organische Reste, die keinen Zerewitinoff-aktiven Wasserstoff aufweisen
sind.

In der Formel (V) können die Reste R₁₆, R₁₇, R₁₈ jeweils unabhängig voneinander lineare oder verzweigte, insbesondere gesättigte, aliphatische C1 bis C12, bevorzugt C2 bis C10, besonders bevorzugt C3 bis C8 und ganz besonders bevorzugt C3 bis C6 Kohlenwasserstoffreste sein. Derartige aminofunktionelle Verbindungen zeichnen sich dadurch aus, dass sie besonders schnell mit dem Prepolymer zu einem stark klebenden, elastischen und festen Polyharnstoff-Netzwerk aushärten.

Die Reste R₁₄, R₁₅ können jeweils unabhängig voneinander lineare oder verzweigte organische C1 bis C 10, bevorzugt C1 bis C8, besonders bevorzugt C2 bis C6, ganz besonders bevorzugt C2 bis C4 Reste und insbesondere aliphatische Kohlenwasserstoffreste. Auch diese Verbindung zeichnet sich durch eine schnelle Netzwerkbildung bei einer Umsetzung mit dem Prepolymer aus.

Bevorzugt ist ebenfalls, wenn jeweils die Reste R₁₄ und R₁₅ gleich und / oder jeweils die Reste R₁₆, R₁₇, R₁₈ gleich sind.

Ganz besonders bevorzugt ist eine Verbindung der Formel (V), bei der R₁₄ und R₁₅ jeweils gleichzeitig Methyl oder Ethyl und R₁₆, R₁₇, R₁₈ jeweils gleichzeitig Ethyl, Propyl oder Butyl sind.

Bevorzugt ist auch eine aminofunktionellen Verbindung B) der allgemeinen Formel (VI) 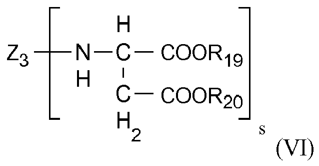 in der
- Z₃: ein organischer Rest, bevorzugt enthaltend eine sekundäre Aminofunktion, ist,
- R₁₉, R₂₀: gleiche oder verschiedene organische Reste sind, die keinen Zerewitinoff-aktiven Wasserstoff aufweisen und
- s: eine ganze Zahl von mindestens 2 ist,

Besonders bevorzugt ist s in der Formel (VI) eine ganze Zahl ≥2 ≤ 4 und ganz besonders bevorzugt gleich 2.

Z₃ kann insbesondere ein Rest der Formel (VII) 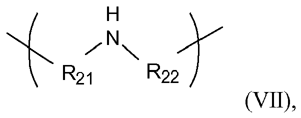 sein, in der
- R₂₁, R₂₂: jeweils unabhängig voneinander ein organischer Rest sind, der keinen Zerewitinoff-aktiven Wasserstoff aufweist.

Besonders bevorzugt ist hier, wenn R₂₁, R₂₂ jeweils unabhängig voneinander oder gleichzeitig ein linearer oder verzweigter, gegebenenfalls auch in der Kette mit Heteroatomen substituierter gesättigter organischer Rest, insbesondere ein linearer oder verzweigter, gesättigter, aliphatischer C1 bis C10, bevorzugt C2 bis C8 und ganz besonders bevorzugt C2 bis C6 Kohlenwasserstoffrest sind. Derartige Polyhamstoff-Systeme härten besonders schnell aus.

Ebenfalls vorteilhaft ist eine Verbindung der Formel (VI) in der die Reste R₁₉, R₂₀ jeweils unabhängig voneinander lineare oder verzweigte C1 bis C 10, bevorzugt C1 bis C8, besonders bevorzugt C2 bis C6, ganz besonders bevorzugt C2 bis C4 organische Reste und insbesondere aliphatische Kohlenwasserstoffreste sind. Beispiele besonders geeigneter Reste sind Methyl, Ethyl, Propyl und Butyl.

Der erfindungsgemäße Schichtaufbau kann leicht von einem Chirurgen verwendet werden, um damit schnell, sicher und zuverlässig Gewebeleckagen, aus denen ein Fluid austritt, zu verschließen. Dazu kann er den Schichtaufbau mit der Klebstoffschicht auf das die Leckage umgebende Gewebe drücken und hier solange fixieren, bis das Gemisch der Klebstoffschicht ausreichend ausgehärtet ist, um die Leckage endgültig abzudichten. Das erfindungsgemäß in der Klebstoffschicht enthaltene Gemisch der Komponenten A) und B) gewährleistet dabei eine schnelle Aushärtung und eine hohe mechanische Belastbarkeit. Die Trägerfolie trägt zur anfänglichen Abdichtung der Leckage bei und ermöglicht es darüber hinaus, den Klebstoff im Bereich der Leckage zu fixieren, bis er ausgehärtet ist.

Das isocyanatfunktionelle Prepolymer A) kann durch Umsetzung eines Polyisocyanats A1) mit einem Polyol A2) gegebenenfalls unter Zusatz von Katalysatoren sowie Hilfs- und Zusatzstoffen hergestellt werden.

Gemäße einer besonders bevorzugten Ausführungsform des erfindungsgemäßen Schichtaufbaus ist das Polyisocyanat A1) wenigstens eine Verbindung aus der Gruppe 1,4-Butylendiisocyanat, 1,6-Hexamethylendiisocyanat, Isophorondiisocyanat, 2,2,4- und / oder 2,4,4-Trimethylhexa-methylendiisocyanat, die isomeren Bis-(4,4'-isocyanatocyclohexyl)-methane oder deren Mischungen beliebigen Isomerengehalts, 1,4-Cyclohexylendüsocyanat, 4-Isocyanatomethyl-1,8-octandiisocyanat, Alkyl-2,6-düsocyanatohexanoat mit C1-C8-Alkylgruppen ist.

Ganz besonders bevorzugt wird Hexamethylendiisocyanat als Polyisocyanat A1) eingesetzt.

Neben den vorstehend genannten monomeren Polyisocyanaten A1) können auch deren höhermolekulare Folgeprodukte mit Uretdion-, Isocyanurat-, Urethan-, Allophanat-, Biuret-, Iminooxadiazindion- oder Oxadiazintrionstruktur sowie deren Mischungen eingesetzt werden. Bevorzugt sind Polyisocyanate A1) der vorstehend genannten Art mit ausschließlich aliphatisch oder cycloaliphatisch gebundenen Isocyanatgruppen oder deren Mischungen.

Ebenfalls bevorzugt ist, wenn Polyisocyanate A1) der vorstehenden Art mit einer mittlere NCO-Funktionalität von 1,5 bis 2,5, bevorzugt von 1,6 bis 2,4, weiter bevorzugt von 1,7 bis 2,3, ganz besonders bevorzugt von 1,8 bis 2,2 und insbesondere von 2 verwendet werden.

Gemäß einer weiteren bevorzugten Ausführungsform können die Polyole A2) Polyesterpolyole und / oder Polyester-Polyether-Polyole und / oder Polyetherpolyole sein. Insbesondere bevorzugt sind dabei Polyester-Polyether-Polyole und / oder Polyetherpolyole mit einem Ethylenoxidanteil zwischen 60 und 90 Gew.-%.

Bevorzugt ist auch, wenn die Polyole A2) ein zahlenmittleres Molekulargewicht von 4000 bis 8500 g/mol aufweisen.

Geeignete Polyetheresterpolyole werden entsprechend dem Stand der Technik vorzugsweise durch Polykondensation aus Polycarbonsäuren, Anhydriden von Polycarbonsäuren, sowie Estern von Polycarbonsäuren mit leichtflüchtigen Alkoholen, bevorzugt C1 bis C6 Monoolen, wie Methanol, Ethanol, Propanol oder Butanol, mit molar überschüssigem, niedermolekularem und/oder höhermolekularem Polyol hergestellt, wobei als Polyol ethergruppenhaltige Polyole gegebenenfalls in Mischungen mit anderen ethergruppenfreien Polyolen eingesetzt werden können.

Selbstverständlich können zur Polyetherestersynthese auch Gemische der höhermolekularen und der niedermolekularen Polyole verwendet werden.

Solche molar überschüssigen niedermolekularen Polyole sind Polyole mit Molmassen von 62 bis 299 Dalton, mit 2 bis 12 C-Atomen und Hydroxylfunktionalitäten von mindestens 2, die weiterhin verzweigt oder unverzweigt sein können und deren Hydroxylgruppen primär oder sekundär sind. Diese niedermolekularen Polyole können auch Ethergruppen aufweisen. Typische Vertreter sind Ethylenglykol, Propandiol-1,2, Propandiol-1,3, Butandiol-1,4, Butandiol-2,3, 2-Methyl-propandiol-1,3, Pentandiol-1,5, Hexandiol-1,6, 3-Methyl-pentandiol-1,5, 1,8-Octandiol, 1,10-Decandiol, 1,12-Dodecandiol, Cyclohexandiol, Diethylenglykol, Triethylenglykol und höhere Homologe, Dipropylenglykol, Tripropylenglykol und höhere Homologe, Glycerin, 1,1,1-Trimethylolpropan, sowie Oligo-tetrahydrofurane mit Hydroxylendgruppen. Selbstverständlich können auch Gemische verwendet werden.

Molar überschüssige höhermolekulare Polyole sind Polyole mit Molmassen von 300 bis 3000 Dalton, die durch ringöffenende Polymerisation von Epoxiden, bevorzugt Ethylen- und / oder Propylenoxid, sowie durch säurekatalysierte, ringöffnende Polymerisation von Tetrahydrofuran, erhalten werden können. Zur ringöffnenden Polymerisation von Epoxiden können entweder Alkalihydroxide oder Doppelmetallcyanidkatalysatoren verwendet werden.

Als Starter für ringöffnende Epoxidpolymerisationen können alle mindestens bifunktionellen Moleküle aus der Gruppe der Amine und der oben genannten niedermolekularen Polyole verwendet werden. Typische Vertreter sind 1,1,1-Trimethylolpropan, Glycerin, o-TDA, Ethylendiamin, Propylenglykol-1,2, etc. sowie Wasser, einschließlich deren Gemische. Selbstverständlich können innerhalb der Gruppe der überschüssigen höhermolekularen Polyole auch Gemische verwendet werden.

Der Aufbau der höhermolekularen Polyole, soweit es sich um Hydroxylgruppen terminierte Polyalkylenoxide aus Ethylen- und/oder Propylenoxid handelt, kann statistisch oder blockweise erfolgen, wobei auch Mischblöcke enthalten sein können.

Polycarbonsäuren sind sowohl aliphatische als auch aromatische Carbonsäuren, die sowohl cyclisch, linear, verzweigt oder unverzweigt sein können und die zwischen 4 und 24 C-Atome aufweisen können.

Beispiele sind Bernsteinsäure, Glutarsäure, Adipinsäure, Azelainsäure, Sebacinsäure, 1,10-Decandicarbonsäure, 1,12-Dodecandicarbonsäure, Phthalsäure, Terephthalsäure, Isophthalsäure, Trimellitsäure, Pyromellitsäure. Bevorzugt sind Bernsteinsäure, Glutarsäure, Adipinsäure, Sebacinsäure, Milchsäure, Phthalsäure, Terephthalsäure, Isophthalsäure, Trimellitsäure, Pyromellitsäure. Besonders bevorzugt sind Bernsteinsäure, Glutarsäure und Adipinsäure.

Weiterhin umfasst die Gruppe der Polycarbonsäuren auch Hydroxycarbonsäuren, bzw. deren innere Anhydride, wie z.B. Caprolacton, Milchsäure, Hydroxybuttersäure, Ricinolsäure, usw. Mit umfasst sind weiterhin auch Monocarbonsäuren, insbesondere solche, die über mehr als 10 C-Atome verfügen, wie Sojaölfettsäure, Palmölfettsäure und Erdnussölfettsäure, wobei deren Anteil am gesamten, das Polyetheresterpolyol aufbauenden Reaktionsmischung 10 Gew.-% nicht übersteigt und zusätzlich die dadurch einhergehende Minderfunktionalität durch Mitverwendung von mindestens trifunktionellen Polyolen, sei es auf Seiten der niedermolekularen oder der hochmolekularen Polyole ausgeglichen wird.

Die Herstellung der Polyetheresterpolyol erfolgt entsprechend dem Stand der Technik bei erhöhter Temperatur im Bereich von 120 bis 250 °C, zunächst unter Normaldruck, später unter Anlegen von Vakuum von 1 bis 100 mbar, vorzugsweise, aber nicht notwendigerweise unter Verwendung eines Veresterungs- oder Umesterungskatalysators, wobei die Reaktion so weit vervollständigt wird, dass die Säurezahl auf Werte von 0,05 bis 10 mg KOH/g, bevorzugt 0,1 bis 3 mg KOH/g und besonders bevorzugt 0,15 bis 2,5 mg KOH/g absinkt.

Weiterhin kann im Rahmen der Normaldruckphase vor dem Anlegen von Vakuum ein Inertgas verwendet werden. Selbstverständlich können alternativ oder für einzelne Phasen der Veresterung auch flüssige oder gasförmige Schleppmittel zum Einsatz kommen. Beispielsweise kann das Reaktionswasser unter Verwendung von Stickstoff als Trägergas, ebenso ausgetragen werden, wie unter Einsatz eines Azeotropschleppmittels, wie z.B. Benzol, Toluol, Xylol, Dioxan, etc.

Selbstverständlich können auch Abmischungen von Polyetherpolyolen mit Polyesterpolyolen in beliebigen Verhältnissen eingesetzt werden.

Polyetherpolyole sind bevorzugt Polyalkylenoxid-Polyether auf Basis von Ethylenoxid und gegebenenfalls Propylenoxid.

Diese Polyetherpolyole basieren bevorzugt auf di- oder höherfunktionellen Startermolekülen wie zwei- oder höherfunktionellen Alkoholen oder Aminen.

Beispiele solcher Starter sind Wasser (als Diol aufgefasst), Ethylenglykol, Propylenglykol, Butylenglykol, Glycerin, TMP, Sorbit, Pentaerythrit, Triethanolamin, Ammoniak oder Ethylendiamin.

Ebenfalls können Hydroxylgruppen aufweisende Polycarbonate, bevorzugt Polycarbonatdiole, mit zahlenmittleren Molekulargewichten Mₙ von 400 bis 8000 g/mol, bevorzugt 600 bis 3000 g/mol eingesetzt werden. Diese sind durch Reaktion von Kohlensäurederivaten, wie Diphenylcarbonat, Dimethylcarbonat oder Phosgen, mit Polyolen, bevorzugt Diolen, erhältlich.

Beispiele derartiger Diole sind Ethylenglykol, 1,2- und 1,3-Propandiol, 1,3- und 1,4-Butandiol, 1,6-Hexandiol, 1,8-Octandiol, Neopentylglykol, 1,4-Bishydroxymethylcyclohexan, 2-Methyl-1,3-propandiol, 2,2,4-Trimethylpentandiol-1,3, Dipropylenglykol, Polypropylenglykole, Dibutylen-glykol, Polybutylenglykole, Bisphenol A und lactonmodifizierte Diole der vorstehend genannten Art in Frage.

Zur Herstellung des Prepolymers A) kann das Polyisocyanat A1) mit dem Polyol A2) bei einem NCO/OH-Verhältnis von bevorzugt 4:1 bis 12:1, besonders bevorzugt 8:1 umgesetzt und anschließend der Anteil an nicht umgesetzten Polyisocyanates mittels geeigneter Methoden abgetrennt werden. Üblicherweise wird hierfür die Dünnschichtdestillation verwendet, wobei Prepolymere mit Restmonomergehalten von weniger als 1 Gew.-%, bevorzugt weniger als 0,1 Gew.-%, ganz besonders bevorzugt weniger als 0,03 Gew.-% erhalten werden.

Gegebenenfalls können während der Herstellung Stabilisatoren wie Benzoylchlorid, Isophthaloylchlorid, Dibutylphosphat, 3-Chlorpropionsäure oder Methyltosylat zugesetzt werden.

Die Reaktionstemperatur bei der Herstellung der Prepolymere A) beträgt dabei bevorzugt 20 bis 120 °C und weiter bevorzugt 60 bis 100 °C.

Die hergestellten Prepolymere haben einen nach DIN EN ISO 11909 gemessenen mittleren NCO-Gehalt von 2 bis 10 Gew.-%, bevorzugt 2,5 bis 8 Gew.-%.

Gemäß einer weiteren Ausführungsform können die Prepolymere A) eine mittlere NCO-Funktionalität von 1,5 bis 2,5, bevorzugt von 1,6 bis 2,4, weiter bevorzugt von 1,7 bis 2,3, ganz besonders bevorzugt von 1,8 bis 2,2 und insbesondere von 2 aufweisen.

Gemäß einer besonders bevorzugten Ausführungsform des erfindungsgemäßen Schichtaufbaus ist vorgesehen, dass das Prepolymer A) und / oder die aminofunktionelle Verbindung B) wenigstens eine unter physiologischen Bedingungen spaltbare Gruppe aufweisen. In diesem Fall ist der Klebstoff der Klebstoffschicht biologisch abbaubar.

Bei der spaltbare Gruppe kann es sich insbesondere um eine Acetal- oder eine Estergruppe handeln. Diese Gruppen ermöglichen einen besonders schnellen biologischen Abbau unter physiologischen Bedingungen.

Idealerweise bleibt das Material solange erhalten, bis die Wundheilung abgeschlossen ist und die mechanische Beanspruchung und die abdichtende Wirkung wieder vom Gewebe übernommen werden kann. Über die Anzahl bzw. Konzentration der abbaubaren Gruppe und der Netzwerkstruktur des ausgehärteten Materials lässt sich der Abbauzeitraum je nach Bedarf einstellenDas Gemisch zur Herstellung der Klebstoffschicht kann weiterhin als Komponente C) Wasser und/oder tertiäres Amin enthalten.

So wurde festgestellt, dass bereits durch die alleinige Zugabe von entweder Wasser oder von tertiärem Amin die Umsetzungsgeschwindigkeit des Prepolymers A) mit der Komponente B) deutlich erhöht und damit die Aushärtzeit vermindert wird. Überraschender Weise steigt die Aushärtungszeit noch stärker an, wenn sowohl Wasser als auch tertiäres Amin zusammen zugesetzt werden. So wird die Umsetzungsgeschwindigkeit noch einmal um das ca. 1, 3 fache erhöht, wenn eine Kombination von Wasser und tertiärem Amin an Stelle einer entsprechenden Menge nur einer der beiden Substanzen verwendet wird.

Gemäß einer bevorzugten Ausführungsform enthält das Gemisch tertiäre Amine der allgemeinen Formel 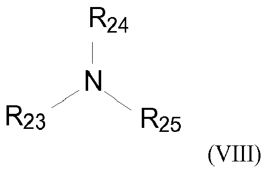 wobei R₂₃, R₂₄, R₂₅ unabhängig voneinander Alkyl- oder Heteroallcyreste mit Heteroatomen in der Alkylkette oder an deren Ende sein können, oder R₅ und R₆ gemeinsam mit dem sie tragenden Stickstoffatom einen aliphatischen, ungesättigten oder aromatischen Heterozyklus bilden können, der gegebenenfalls weitere Heteroatome enthalten kann.

Besonders bevorzugt ist, wenn das tertiäre Amin aus der Gruppe Triethanolamin, Tetrakis (2-hydroxyethyl) ethylendiamin, N,N-Dimethyl-2-(4-methylpiperazin-1-yl)ethanamin, 2-{[2-(Dimethylamino)ethyl](methyl)amino}ethanol sowie 3,3',3"-(1,3,5-Triazinan-1,3,5-triyl)tris(N,N-dimethyl-propan-1-amin) ausgewählt ist.

Die Konzentration des Wassers im Gemisch kann insbesondere 0,2 bis 2,0 Gew.-% betragen. Bei der Zugabe von 0,2 Gew.-% Wasser erhöht sich die durchschnittliche Umsetzungsgeschwindigkeit um den Faktor 2. Wenn 2,0 Gew.-% Wasser zugesetzt werden, tritt eine Erhöhung der Umsetzungsgeschwindigkeit um den Faktor 8 auf.

Dem Gemisch können insbesondere 0,1 bis 1,0 Gew.-% tertiäres Amin zugesetzt werden. Dabei bewirkt ein Zusatz von 0,1 Gew.-% eine Verdoppelung der durchschnittlichen Umsetzungsgeschwindigkeit und die Zugabe von 1,0 Gew.-% führt zu einer Erhöhung um den Faktor 2,5.

Weiterhin ist es möglich, dass das Gemisch als Komponente D) pharmakologisch aktive Verbindungen, insbesondere Analgetika mit oder ohne antiinflammatorische Wirkung, Antiphlogistika, antimikrobiell wirksame Substanzen oder Antimykotika umfasst.

Unter pharmakologisch aktiven Verbindungen werden allgemein Stoffe und Zubereitungen aus Stoffen, die zur Anwendung am oder im menschlichen oder tierischen Körper bestimmt sind, um Krankheiten, Leiden, Körperschäden oder krankhafte Beschwerden zu heilen, zu lindern, zu verhüten oder zu erkennen verstanden. Darunter fallen ebenfalls Stoffe und Zubereitungen daraus, um Krankheitserreger, Parasiten oder körperfremde Stoffe abzuwehren, zu beseitigen oder unschädlich zu machen.

Analgetika sind schmerzstillende Substanzen unterschiedlicher chemischer Struktur und Wirkungsweise.

Antiphlogistika sind entzündungshemmende Substanzen.

Antimikrobiell wirksame Substanzen / Antiseptika sind Verbindungen, die das Wachstum bestimmter Mikroorganismen wie z. B. Bakterien, Pilze, Algen und Protozoen inhibiert und/oder diese absterben lässt.

Antimykotika sind Arzneimittel zur Behandlung von Pilzinfektionen.

Antiparasitär wirksame Verbindungen sind Wirkstoffe, die Parasiten abtöten oder deren Wachstum sowie das Einnisten in menschlichem oder tierischem Gewebe inhibieren bzw. verhindern.

Die pharmakologisch aktive Verbindung sollte bevorzugt bei Raumtemperatur in der Komponente B) löslich sein, kann aber auch in B) suspendiert eingesetzt werden.

Die Konzentration der pharmakologisch aktive Verbindung richtet sich nach den therapeutisch notwendigen Dosen und liegt im allgemeinen bei 0,001 Gew.-% bis 10 Gew.-%, bevorzugt bei 0,01 Gew.-% bis 5 Gew.-% bezogen auf die Gesamtmenge aller nichtflüchtigen Komponenten des Gemisches.

Geeignete Analgetika sind Lokalanästhetika wie Ambucain, Amylocain, Arecaidin, Benoxinat, Benzocain, Betoxycain, Butacain, Butethamin, Bupivacain, Butoxycain, Chlorprocain, Cocaethylen, Cocaine, Cyclomethycain, Dibucain, Dimethocain, Dimethisoquin, Etidocain, Fomacain, Isobutyl-p-aminobenzoat, Leucinocain, Lidocain, Meperidin, Mepivacain, Metabutoxycain, Octacain, Orthocain, Oxethazain, Phenacain, Piperocain, Piridocain, Pramoxin, Procain, Procainamid, Proparacain, Propoxycain, Pseudococain, Pyrrocain, Ropivacain, Tetracain, Tolycain, Tricain, Trimecain, Tropacocain, Amolanon, Cinnamoylcocain, Paretoxycain, Propiocain, Myrtecain und Propanocain.

Ebenso eingesetzt werden können Opioid-Analgetika wie Morphin und seine Derivate (z. B. Codein, Diamorphin, Dihydrocodein, Hydromorphon, Oxycodon, Hydrocodon, Buprenorphin, Nalbuphin, Pentazocin), Pethidin, Levomethadon, Tilidin und Tramadol.

Gleichfalls können nichtsteroidale Antiphlogistika (NSAID) wie Acetylsalicylsäure, Acemetacin, Dexketoprofen, Diclofenac, Aceclophenac, Diflunisal, Piritramid, Etofenamat, Felbinac, Flurbiprofen, Flufeaminsäure, Ibuprofen, Indometacin, Ketoprofen, Lonazolac, Lomoxicam, Mefenaminsäure, Meloxicam, Naproxen, Piroxicam, Tiaprofensäure, Tenoxicam, Phenylbutazon, Propyphenazon, Phenazon und Etoricoxib verwendet werden. Andere Analgetika wie Azapropazon, Metamizol, Nabumeton, Nefopam, Oxacephrol, Paracetamol sowie das analgetisch wirksame Amitriptylin können selbstverständlich ebenfalls eingesetzt werden.

Neben den genannten Analgetika, die eine entzündungshemmende Wirkung haben, können zusätzlich rein antiphlogistisch wirksame Verbindungen verwendet werden. Dazu gehört die Klasse der Glucocorticoide wie z. B. Cortison, Betamethason, Dexamethason, Hydrocortison, Methylprednisolon, Prednisolon, Prednison, Budesonid, Allotetrahydro-cortison, Fludrocortison, Fluprednisolon, Fluticasonpropionat, etc.

Als antiseptisch wirksame Substanzen können unter anderem die folgenden Verbindungen eingesetzt werden: Triclosan (2,4,4'-Trichloro-2'hydroxydiphenyl ether), Chlorhexidin und seine Salze, Octenidin, Chloramphenicol, Florfenicol, Chlorchinaldol, Iod, Povidon-Iod, Hexachlorophen, Merbromin, PHMB, nanokristallin vorliegendes Silber sowie Silber- und Kupfersalze.

Des weiteren können als antimikrobiell wirksame Substanzen Antibiotika aus der Klasse der β-Lactame (z. B. Penicillin und seine Derivate, Cephalosporine), der Tetracycline (z. B. Demeclocyclin, Doxycyclin, Oxytetracycline, Minocyclin, Tetracyclin), der Makrolide (z. B. Erythromycin, Josamycin, Spiramycin), der Lincosamide (z. B. Clindamycin, Lincomycin), der Oxazolidinone (z. B. Linezolid), der Gyrasehemmer (z. B. Danofloxacin, Difloxacin, Enrofloxacin, Ibafloxacin, Marbofloxacin, Nalidixinsäure, Pefloxacin, Fleroxacin, Levofloxacin) und der zyklischen Peptide (z. B. Bicozamycin) verwendet werden. Ebenso eingesetzt werden können Rifamycin, Rifaximin, Methenamin; Mupirocin, Fusidinsäure, Flumechin, und die Derivate des Nitroimidazols (z. B. Metronidazol, Nimorazol, Tinidazol), des Nitrofurans (Furaltadon, Nifurpirinol, Nihydrazone, Nitrofurantoin), des Sulfonamids (z. B. Sulfabromomethazin, Sulfacetamid, Sulfachlorpyridazine, Sulfadiazine etc.) sowie β-Lactamase-Inhibitoren wie die Clavulansäure.

Als antimykotisch wirksame Substanzen können alle Azolderivate, die die Biosynthese von Ergosterol hemmen, wie z. B. Clotrimazol, Fluconazol, Miconazol, Bifonazol, Econazol, Fenticonazol, Isoconazol, Oxiconazol etc. zum Einsatz kommen. Sonstige lokal anwendbare Antimykotika sind Amorolfin, Ciclopirox, Thymol und seine Derivate sowie Naftifin. Ebenso kann die Klasse der Alkylparabene verwendet werden.

Zu den geeigneten antiparasitär wirksamen Verbindungen gehören unter anderem die Ektoparasitizide Cyfluthrin und Lindan, verschiedene Azolderivate wie z. B. Dimetridazol und Metronidazol sowie Chinin.

In Weiterbildung der Erfindung ist vorgesehen, dass die Trägerfolie aus einem bioabbaubaren Material besteht. Beispiele für geeignete bioabbaubare Materialien sind Gelatine, Chitosan, Cellulose, Polylactid, Polyglycolid, Polycaprolacton oder Copolymere bzw. Mischungen aus den genannten Stoffen.

Besonders bevorzugt ist, wenn gleichzeitig das Prepolymer A) und / oder die aminofunktionelle Verbindung B) wenigstens eine unter physiologischen Bedingungen spaltbare Gruppe aufweist, da dann der gesamte Schichtaufbau unter physiologischen Bedingungen abbaubar ist. Dies hat den Vorteil, dass nach erfolgter Ausheilung keine körperfremden Materialien zurückbleiben.

Im Rahmen der vorliegenden Erfindung wird unter einer unter physiologischen Bedingungen spaltbaren Gruppe eine funktionelle Gruppe eines Moleküls verstanden, die im menschlichen oder tierischen Körper in wenigstens zwei Teile gespalten werden kann.

Gemäß einer ganz besonders bevorzugten Ausführungsform des erfindungsgemäßen Schichtaufbaus umfasst oder besteht die Trägerfolie ein Polymer, das durch Aushärtung eines Gemisches wenigstens umfassend das Prepolymer A) und die aminofünktionelle Verbindung B) erhältlich ist. Die in diesem Fall vorliegende chemische Ähnlichkeit des Materials der Tragerfolie zu dem Klebstoff der Klebstoffschicht führt zu einer besonders hohen Haftung zwischen der Trägerfolie und der Klebstoffschicht. Des Weiteren sind während der Implantationsphase weniger Komplikationen zu erwarten, da der menschliche Körper sich nur mit einem homogenen Fremdkörper konfrontiert wird und damit die Fremdkörperreaktion milder verlaufen kann.

Die Trägerfolie kann insbesondere eine Dicke im Bereich von 100 µm bis 2000 µm, bevorzugt von 200 µm bis 1500 µm, weiter bevorzugt von 300 µm bis 1000 µm und besonders bevorzugt von 400 µm bis 800 µm aufweisen

Die Klebstoffschicht kann insbesondere eine Dicke im Bereich von 0,5 mm bis 8 mm, bevorzugt von 0,6 mm bis 7 mm, weiter bevorzugt von 0,7 mm bis 6 mm und besonders bevorzugt von 0,8 mm bis 5 mm aufweisen

Bevorzugt ist auch, wenn die Klebstoffschicht eine Oberfläche der Trägerfolie vollständig bedeckt. In diesem Fall wird eine besonders gute Haftung des Schichtaufbaus am Gewebe sicher gestellt. Alternativ können aber auch nur einzelne Bereiche der Trägerfolie mit der Klebeschicht bedeckt sein.

Der erfindungsgemäße Schichtaufbau ist besonders zum Verschließen von Gewebeleckagen geeignet. Er eignet sich besonders dazu, blutende Gefäße zu verschließen und so die Blutung zu stillen. Es ist ebenfalls möglich, Leckagen in Gefäßen zu verschließen, die andere Flüssigkeiten oder Gase wie Luft führen.

Ein weiterer Gegenstand der Erfindung ist ein Verfahren zur Herstellung eines erfindungsgemäßen Schichtaufbaus bei dem das Gemisch durch Vermengen des Prepolymers A) mit der aminofunktionellen Verbindung B) und gegebenenfalls weiterer Komponenten hergestellt und zumindest bereichsweise auf die Trägerfolie aufgebracht wird.

Ebenfalls Gegenstand der Erfindung ist ein Kit zur Herstellung eines erfindungsgemäßen Schichtverbunds umfassend eine erste Klebstoffkomponente, eine zweite Klebstoffkomponente und eine Trägerfolie, wobei die erste Klebstoffkomponente das Prepolymer A) und die zweite Klebstoffkomponente die aminofunktionelle Verbindung B) und gegebenenfalls weitere Komponenten umfasst.

Die Erfindung wird im Folgenden anhand von Beispielen näher erläutert.

### Methoden

- Säurezahl / OH-Zahl:: Die Säure- bzw. OH-Zahl wurde nach DIN ISO 17025 bestimmt
- NCO-Gehalt:: Der NCO-Gehalt wurde, wenn nicht ausdrücklich anders erwähnt, volumetrisch gemäß DIN-EN ISO 11909 bestimmt
- Viskosität:: Die Viskosität wurde nach ISO 3219 bei 23°C bestimmt.
- Restmonomerengehalt:: Der Restmonomerengehalt wurde nach DIN ISO 17025 bestimmt.

### Substanzen

- PEG 600:: Polyethylenglykol mit dem zahlenmittleren Molekulargewicht von 600 g/Mol
- PEG 800:: Polyethylenglykol mit dem zahlenmittleren Molekulargewicht von 800 g/Mol

### Synthese der aminofunktionellen Verbindung Tetraethyl-2,2'-[(2-methylpentan-1,5-diyl)diimino]dibutandioat (1):

Zu 2 Mol Diethylmaleat wurde unter Stickstoffatmosphäre langsam 1 Mol 2-Methyl-1,5-diaminopentan getropft, so dass die Reaktionstemperatur 60 °C nicht überschritt. Anschließend wurde so lange auf 60 °C erwärmt, bis kein Diethylmaleat mehr im Reaktionsgemisch nachweisbar war (mittels ¹H-NMR verifiziert; charakteristisches Singulet bei 6,24 ppm in CDCl₃ verschwand) Das Produkt wurde quantitativ als gelbe Flüssigkeit erhalten.

### Synthes der aminofunktionellen Verbindung Triethyl-4-oxo-3-oxa-7,14,21-triazatricosan-6,22,23-tricarboxylat (2):

Zu 154 g (0,72 Mol) Bis(hexamethylen)-triamin wurden 246 g (1,42 Mol) Maleinsäurediethylester gegeben. Die Reaktionsmischung wurde 3 Tage bei 60 °C gerührt. Das Produkt wurde quantitativ als gelbe Flüssigkeit erhalten.

### Polyol A)

Aus einer gerührten Mischung bestehend aus 24,7 g Adipinsäure und 281,4 g PEG 600 wurden über 12 h bei 210-220 °C bei Atmosphärendruck Wasser abdestilliert. Anschließend wurden 30 mg Zinn(II)chlorid der Mischung zugegeben und unter Vakuum für weitere 24 h Wasser abdestilliert, bis das Gemisch eine Säurezahl kleiner als 1 aufwies.

### Polyol B)

Aus einer gerührten Mischung bestehend aus 212,55 g Bernsteinsäure und 1591,56 g PEG 600 wurden über 12 h bei 210-220 °C bei Atmosphärendruck Wasser abdestilliert. Anschließend wurden 180 mg Zinn(II)chlorid der Mischung zugegeben und unter Vakuum für weitere 24 h Wasser abdestilliert, bis das Gemisch eine Säurezahl kleiner als 1 aufwies.

### Polyol C)

Aus einer gerührten Mischung bestehend aus 49,29 g Bernsteinsäure und 519,6 g PEG 800 wurden über 12 h bei 210-220 °C bei Atmosphärendruck Wasser abdestilliert. Anschließend wurden 50 mg Zinn(II)chlorid der Mischung zugegeben und unter Vakuum für weitere 24 h Wasser abdestilliert, bis das Gemisch eine Säurezahl kleiner als 1 aufwies.

### Prepolymer PA)

336 g 1,6-Hexamethylendiisocyanat (HDI) wurden mit 0,1 Gew.-% Benzoylchlorid vorgelegt und auf 80 °C erhitzt. Anschließend wurden unter Rühren 197,7 g des zuvor hergestellten Polyesters A) über 1h zudosiert und bis zum Erreichen eines konstanten NCO-Gehalts bei 80 °C weiter gerührt. Das überschüssige HDI wurde bei 140 °C und 0,13 mbar mittels Dünnschichtverdampfer entfernt. Das erhaltene Prepolymer hatte einen NCO-Gehalt von 5,8 % und eine Viskosität von 1010 mPas/23°C. Der Restmonomerengehaltbetrug < 0,03 % HDI.

### Prepolymer PB)

672 g HDI wurden mit 0,1 Gew.-% Benzoylchlorid vorgelegt und auf 80 °C erhitzt. Anschließend wurden unter Rühren 788 g des zuvor hergestellten Polyesters B) über 1h zudosiert und bis zum Erreichen eines konstanten NCO-Gehalts bei 80 °C weiter gerührt. Das überschüssige HDI wurde bei 140 °C und 0,13 mbar mittels Dünnschichtverdampfer entfernt. Das erhaltene Prepolymer hatte einen NCO-Gehalt von 3,5 % und eine Viskosität von 4800 mPas/23°C. Der Restmonomerengehalt betrug < 0,03 % HDI.

### Prepolymer PC)

252 g HDI wurden mit 0,1 Gew.-% Benzoylchlorid vorgelegt und auf 80 °C erhitzt. Anschließend wurden unter Rühren 307,2 g des zuvor hergestellten Polyesters C) über 1h zudosiert und bis zum Erreichen eines konstanten NCO-Gehalts bei 80 °C weiter gerührt. Das überschüssige HDI wurde bei 140 °C und 0,13 mbar mittels Dünnschichtverdampfer entfernt. Das erhaltene Prepolymer hatte einen NCO-Gehalt von 2,8 % und eine Viskosität von 6460 mPas/23°C. Der Restmonomerengehalt betrug < 0,03 % HDI.

### Trägerschicht FPA)

4 g Prepolymer PA wurden mit 1,32 g **1** vermischt und auf einer PE-Folie jeweils mit einem Rakel als 500 µm und 750 µm dicke Schichten aufgetragen und 1 Tag aushärten gelassen. Nach dem Aushärten ließ sich die gerakelte Schciht von der PE-Folie abziehen und als bioabbaubares Trägerschicht einsetzen.

### Trägerschicht FPB)

4 g Prepolymer PB wurden mit 1,32 g **1** vermischt und auf einer PE-Folie jeweils mit einem Rakel als 500 µm und 750 µm dicken Schichten aufgetragen und 1 Tag aushärten gelassen. Nach dem Aushärten ließ sich die gerakelte Schicht von der PE-Folie abziehen und als bioabbaubares Trägerschicht einsetzen.

### Trägerschicht FPC)

4 g Prepolymer PC wurden mit 0,78 g **1** vermischt und auf einer PE-Folie jeweils mit einem Rakel als 500 µm und 750 µm dicke Schichten aufgetragen und 1 Tag aushärten gelassen. Nach dem Aushärten ließ sich die gerakelte Schicht von der PE-Folie abziehen und als bioabbaubares Trägerschicht einsetzen.

### Bestimmung der Abbauzeit

Die Bioabbaubarkeit der oben hergestellten Trägerschichten wurde wie folgt bestimmt:
Die gerakelten und ausgehärteten Trägerschichten wurden in 0,5 cm breite und 4 cm lange Streifen geschnitten und in saliner PBS Pufferlösung (pH 7,4; Sigma Aldrich) bei 37 °C im Schüttelinkubator gelagert und täglich verifiziert. Eine Probe galt als abgebaut, wenn kein Festkörper mehr zu beobachten war.

Die Ergebnisse der Bestimmung sind in der Tabelle 1 aufgeführt. Spätestens nach 60 Tagen waren alle Trägerschichten vollständig abgebaut.

**Tabelle 1: Bioabbauversuche (in vitro)**

| **Trägerschicht** | **Dicke** | **Abbauzeit bei 60°C in isotonischer NaCl-Lösung** | **Abbauzeit bei 37°C in pH 7,4 PBS Puffer** |
|---|---|---|---|
| FPA | 500 µm | 30 d | 60 d |
| FPA | 750 µm | 30 d | 60 d |
| FPB | 500 µm | 16 d | 8 d |
| FPB | 750 µm | 16 d | 8 d |
| FPC | 500 µm | 16 d | 3 d |
| FPC | 750 µm | 16 d | 7 d |

### In vivo Versuch am Hausschwein mit nicht abbaubarer Trägerfolie

### Versiegelung einer Lungenfistel

Ein ca. 4 cm großes Stück der Lunge wurde reseziert, wobei eine Lungenfistel entstand. Der Durchmesser des Bronchos betrug ca. 3 mm. Es kam zu einer arteriellen Blutung. Es wurden ca. 3 ml eines Klebstoffs, bestehend aus einer äquivalenten Mischung der Prepolymers PC) und der aminofunktionellen Verbindung **2**), schnell aufgetragen und direkt mit einem Stück Parafilm auf die Wunde gepresst. Der Parafilm wurde 30 s lang aufgedrückt und nach 110 Sekunden entfernt. Der Klebstoff härtete innerhalb von ca. 30 Sekunden aus. Die Inzision war abgedichtet und hielt einem Peak-Druck von 22 mm Hg stand.

### Versiegelung einer Stichverletzung am Herz:

Mit einem Skalpell wurde am Herz eine Stichverletzung erzeugt, wobei es zu einer spritzenden Blutung kam (Verletzung der Koronarvene im linken Ventrikel). Unbehandelt hätte diese innerhalb von 1-2 min zum Tod geführt. Fibrin könnte nicht eingesetzt werden, da es aufgrund seiner niedrigen Viskosität sofort weggespült würde. Nach Auftragen von ca. 3 ml des Klebstoffs, bestehend aus einer äquivalenten Mischung der Prepolymers PC) und der aminofunktionellen Verbindung **2**), wurde ein Stück Parafilm 1 Minute auf die Verletzung gepresst. Der Parafilm ließ sich leicht ablösen. Die Blutung wurde stark verringert, kam jedoch nicht vollständig zum Stillstand. Nach Auftragen weiterer 2 ml des Klebstoffs konnte die Wunde komplett abgedichtet werden und hielt einem Blutdruck von 140 mm Hg stand.

### Applikationsversuche mit nicht abbaubaren Trägerschichten auf Muskelgewebe:

4 g des Prepolymers PC wurden gut mit einer äquivalenten Menge der aminofunktionellen Verbindung **2** in einem Becher verrührt. Der Klebstoff wurde unmittelbar danach auf eine Trägerschicht (siehe Tabelle 2) aufgestrichen und einem Applikationsversuch auf Muskelgewebe unterzogen. Dazu wurde die mit Klebstoff bestrichene Trägerschicht auf ein ca. 5 x 10 cm großes und 1 cm dickes Stück Muskelgewebe gedrückt. Nach der Aushärtung wurde versucht die nicht abbaubare Trägerschicht zu entfernen. In Tabelle 2 sind die eingesetzten Schichten sowie die Ablösbarkeit der Schichten vom ausgehärteten Klebstoff (sehr leicht (---) bis sehr schwer (+++)) dargestellt.

**Tabelle 2: Trägerfolie und Ablösbarkeit nach Aushärtung des Klebstoffs**

| **Folie** | **Ablösbarkeit** |
|---|---|
| Mylar 0,35 Biaxial orientierte Folie aus Polyethylente-rephthalat (DuPont) | ++ |
| Nomex Aramid-Folie (DuPont) | --- |
| Lexan Polycarbonat-Folie (Sabic) | ++ |
| PEEK Polyetherketon-Folie (Evonik) | --- |
| Silikon-Folie (Wacker Chemie) | +++ |
| PE-Folie (Leitz) | ++ |
| Polycarbonat-Folie Bayer Material Science | --- |
| Alufolie (Toppits) | --- |
| Parafilm (Pechiney Plastic Packaging) | +++ |
| Klarsichtfolie (Leitz) | +++ |

### Applikationsversuche mit bioabbaubaren Trägerschichten (FPA, FPB, FPC) auf Muskelgewebe:

4 g des Prepolymers PB bzw. PC wurden mit einer äquivalenten Menge der aminofunktionellen Verbindung **2** in einem Becher gut verrührt. Der Klebstoff wurde unmittelbar danach auf eine Trägerschicht (FPA, FPB, FPC) aufgestrichen und einem Applikationsversuch auf Muskelgewebe unterzogen. Dazu wurde die mit Klebstoffbestrichene Trägerschicht auf ein ca. 5 x 10 cm großes und 1 cm dickes Stück Muskelgewebe gedrückt. Nach der Aushärtung war es nicht möglich, die Trägerschicht mit Klebstoff ohne Beschädigung des Muskelgewebes abzulösen.

## Patentansprüche

1. Schichtaufbau mit einer Trägerfolie und einer Klebstoffschicht, wobei die Klebstoffschicht zumindest bereichsweise mit der Trägerfolie verbunden ist und die Klebstoffschicht ein Gemisch wenigstens folgender Komponenten umfasst:
A) ein isocyanatfunktionelles Prepolymer erhältlich durch Umsetzung von wenigstens
A1) einem aliphatischen Polyisocyanat mit
A2) einem Polyol, das insbesondere ein zahlenmittleres Molekulargewicht von ≥ 400 g/mol und eine mittlere OH-Funktionalität von 2 bis 6 aufweisen kann,
B) eine aminofunktionelle Verbindung der allgemeinen Formel (I) 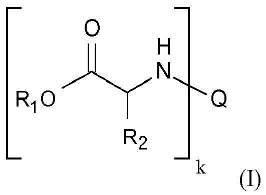 in der
R₁ ein organischer Rest, der keinen Zerewitinoff-aktiven Wasserstoff aufweist,
R₂ ein linearer oder verzweigter, unsubstituierter oder mit Heteroatomen auch in der Kette substituierter organischer Rest ist, der keinen Zerewitinoff-aktiven Wasserstoff aufweist,
Q ein linearer oder verzweigter, unsubstituierter oder mit Heteroatomen auch in der Kette substituierter organischer Rest und
k ≥ 1 und ≤ 6
ist.

2. Schichtaufbau nach Anspruch 1, **dadurch gekennzeichnet, dass** Polyisocyanat A1) wenigstens eine Verbindung aus der Gruppe 1,4-Butylendiisocyanat, 1,6-Hexamethylendiisocyanat, Isophorondiisocyanat, 2,2,4- und / oder 2,4,4-Trimethylhexa-methylendiisocyanat, die isomeren Bis-(4,4'-isocyanatocyclohexyl)-methane oder deren Mischungen beliebigen Isomerengehalts, 1,4-Cyclohexylendiisocyanat, 4-Isocyanatomethyl-1,8-octandüsocyanat, Alkyl-2,6-diisocyanatohexanoat mit C1-C8-Alkylgruppen ist.

3. Schichtaufbau nach einem der Ansprüche 1 oder 2, **dadurch gekennzeichnet, dass** das Polyol A2) wenigstens eine Verbindung aus der Gruppe Polyesterpolyol, Polyester-Polyether-Polyole, Polyetherpolyole und insbesondere ein Polyester-Polyether-Polyol und / oder ein Polyetherpolyol mit einem Ethylenoxidanteil zwischen 60 und 90 Gew.-%, ist.

4. Schichtaufbau nach einem der Ansprüche 1 bis 3, **dadurch gekennzeichnet, dass** das Polyol A2) ein zahlenmittleres Molekulargewicht von 2000 bis 8500 g/mol aufweist.

5. Schichtaufbau nach einem der Ansprüche 1 bis 4, **dadurch gekennzeichnet, dass** das Prepolymer A) und / oder die aminofunktionelle Verbindung B) wenigstens eine unter physiologischen Bedingungen spaltbare Gruppe aufweisen.

6. Schichtaufbau nach Anspruch 5, **dadurch gekennzeichnet, dass** die spaltbare Gruppe eine Acetal- oder eine Estergruppe ist.

7. Schichtaufbau nach einem der Ansprüche 1 bis 6, **dadurch gekennzeichnet, dass** X 4-Methylpentyl, n-Hexamethyl, n-Butyl, Isophoryl, Methyl-bis(cyclohexyl) oder Dihexamethylenylamin ist.

8. Schichtaufbau nach einem der Ansprüche 1 bis 7, **dadurch gekennzeichnet, dass** die Trägerfolie aus einem bioabbaubaren Material besteht.

9. Schichtaufbau nach einem der Ansprüche 1 bis 8, **dadurch gekennzeichnet, dass** die Trägerfolie ein Polymer erhältlich durch Aushärtung eines Gemisches gemäß einem der Ansprüche 1 bis 7 umfasst oder daraus besteht.

10. Schichtaufbau nach einem der Ansprüche 1 bis 9, **dadurch gekennzeichnet, dass** die Trägerfolie eine Dicke im Bereich von 100 µm bis 2000 µm, bevorzugt von 200 µm bis 1500 µm, weiter bevorzugt von 300 µm bis 1000 µm und besonders bevorzugt von 400 µm bis 800 µm aufweist.

11. Schichtaufbau nach einem der Ansprüche 1 bis 10, **dadurch gekennzeichnet, dass** die Klebstoffschicht eine Dicke im Bereich von 0,5 mm bis 8 mm, bevorzugt von 0,6 mm bis 7 mm, weiter bevorzugt von 0,7 mm bis 6 mm und besonders bevorzugt von 0,8 mm bis 5 mm aufweist.

12. Schichtaufbau nach einem der Ansprüche 1 bis 11, **dadurch gekennzeichnet, dass** die Klebstoffschicht eine Oberfläche der Trägerfolie vollständig bedeckt.

13. Schichtaufbau nach einem der Ansprüche 1 bis 12 zum Verschließen von Gewebeleckagen.

14. Verfahren zur Herstellung eines Schichtaufbaus nach einem der Ansprüche 1 bis 13 bei dem das Gemisch durch Vermengen des Prepolymers A) mit der aminofunktionellen Verbindung B) und gegebenenfalls weiterer Komponenten hergestellt und zumindest bereichsweise auf die Trägerfolie aufgebracht wird.

15. Kit zur Herstellung eines Schichtverbunds zum Verschließen von Gewebeleckagen nach einem der Ansprüche 1 bis 12 umfassend eine erste Klebstoffkomponente, eine zweite Klebstoffkomponente und eine Trägerfolie, wobei die erste Klebstoffkomponente das Prepolymer A) und die zweite Klebstoffkomponente die aminofunktionelle Verbindung B) und gegebenenfalls weitere Komponenten umfasst.
